# EUROPEAN PATENT SPECIFICATION

(11) **EP 3 609 566 B1**
(45) Date of publication and mention of the grant of the patent: **26.04.2023**
(21) Application number: 18752990.4
(22) Date of filing: 26.07.2018
(51) Int. Cl.: A61M 25/01, A61M 25/00

(54) **INTRAVASCULAR DELIVERY SYSTEM WITH CENTRALIZED STEERING**
INTRAVASKULÄRES ABGABESYSTEM MIT ZENTRALER LENKUNG
SYSTÈME DE DISTRIBUTION INTRAVASCULAIRE À DIRECTION CENTRALISÉE

(30) Priority: 27.07.2017 US 201715662084
(43) Date of publication of application: 19.02.2020
(73) Proprietor: Evalve Inc., Santa Clara, California 95054 (US)
(72) Inventor: PRABHU, Santosh V., Sunnyvale California 94086 (US); VAN HOVEN, Dylan T., San Carlos California 94070 (US); GADA, Manish B., Santa Clara California 95051 (US)
(74) Representative: Boult Wade Tennant LLP
(86) International application number: PCT/US2018/043929
(87) International publication number: WO 2019/023477

(56) References cited:
- EP-A1- 2 465 568
- WO-A1-2014/182797
- US-A1- 2003 181 855

## Description

### BACKGROUND

The mitral valve controls blood flow from the left atrium to the left ventricle of the heart, preventing blood from flowing backwards from the left ventricle into the left atrium so that it is instead forced through the aorta for distribution throughout the body. A properly functioning mitral valve opens and closes to enable blood flow in one direction. However, in some circumstances the mitral valve is unable to close properly, allowing blood to regurgitate back into the atrium. Such regurgitation can result in shortness of breath, fatigue, heart arrhythmias, and even heart failure.

Mitral valve regurgitation has several causes. Functional mitral valve regurgitation (FMR) is characterized by structurally normal mitral valve leaflets that are nevertheless unable to properly coapt with one another to close properly due to other structural deformations of surrounding heart structures. Other causes of mitral valve regurgitation are related to defects of the mitral valve leaflets, mitral valve annulus, or other mitral valve tissues. In some circumstances, mitral valve regurgitation is a result of infective endocarditis, blunt chest trauma, rheumatic fever, Marfan syndrome, carcinoid syndrome, or congenital defects to the structure of the heart. Other cardiac valves, in particular the tricuspid valve, can similarly fail to properly close, resulting in undesirable regurgitation.

Heart valve regurgitation is often treated by replacing the faulty valve with a replacement valve implant or by repairing the valve through an interventional procedure. However, issues can arise related to deployment and effectiveness of various treatment options. For instance, accessing the targeted treatment site can be challenging. Intravascular delivery of interventional devices can be less invasive relative to open-heart surgery. However, properly routing the necessary equipment through a patient's vasculature, and then properly orienting the interventional components to be able to successfully carry out the procedure, introduces a variety of challenges.

The subject matter claimed herein is not limited to embodiments that solve any disadvantages or that operate only in environments such as those described above. Rather, this background is only provided to illustrate one exemplary technology area where some embodiments described herein may be practiced.

In WO 2014/182797 there is described a medical device, such as an introducer, which includes a shaft having a lumen therethrough. A distal region of the shaft includes both a fixed curve region and a steerable region. According to certain aspects of the disclosure, the fixed curve region is more proximal than the steerable region. The fixed curvature of the distal region is selected to facilitate positioning of the distal end of the medical device in the general vicinity of a target, such as the left atrial appendage, while the steerable region allows for fine tuning. The lumen of the shaft is a large bore, typically on the order of about 12F to about 14F, to facilitate passage of larger medical devices, including left atrial appendage occlusion devices.

In US 2003/181855 there is described a catheter which employs a pre-formed distal end and a proximal deflection mechanism for steering the catheter. A shaped member extends from a shaped region of the catheter sheath to at least a portion of an anchor region. A steering ribbon extends from a proximal region of the sheath and passes within at least a portion of the anchor region. A distal end of the steering ribbon is joined with a proximal portion of the shaped member. At least one steering tendon is disposed within the sheath and has a first end attached at the anchor region and a second end located at the proximal region of the sheath. Movement of the steering tendon in a proximal direction causes the deflection region to deflect relative to the longitudinal axis of the catheter while the shape of shaped region of the sheath is substantially maintained.

In EP 2,465,568 there is described a steerable guide catheter which includes a handle and an elongated shaft extending outwardly away from and coupled to the handle. The shaft has a distal end opposite the handle. The shaft further has a first preformed curved section at the distal end and a second preformed curved section that is located proximal to the first preformed curved section. The two preformed curved sections can be fabricated as part of a molding process whereby the two preformed curved sections are effectively "baked in" the catheter shaft. The guide catheter also has a steering mechanism for controllably steering at least the first preformed curved section at the distal end. The steering mechanism is coupled between the handle and elongated shaft and is configured such that actuation of the steering mechanism causes the first preformed curved section to be bent in at least two planes.

### BRIEF SUMMARY

According to the present invention there is provided a delivery system having the features of claim 1 for delivery of an interventional device in an intravascular procedure.

In one embodiment, an interventional delivery system includes a handle with one or more controls (e.g., knobs, buttons, dials, etc.), and a delivery catheter coupled to the handle. The delivery catheter has a proximal end and a distal end. The proximal end of the delivery catheter is coupled to the handle. The delivery catheter also includes a distal section which is configured to selectively form a compound curve to enable positioning of the distal end relative to a targeted intravascular treatment site. The compound curve includes a first pre-formed curve at a proximal portion of the distal section and a second pre-formed curve at a distal portion of the distal section. A series of control lines are also operatively coupled to a control of the handle and each control line extends from its respective control to toward the distal end of the delivery catheter. The delivery catheter includes a distal coupler at or near its distal end. One or more control lines engage with the distal coupler such that tensioning of the one or more control lines changes position of the distal coupler to form or adjust the compound curve.

In some embodiments, the device is configured such that the first pre-formed curve and the second pre-formed curve of the compound curve lie in different planes. For example, the respective planes in which the first and second pre-formed curves lie may be substantially orthogonal to one another. In some embodiments, the first pre-formed curve is bendable to an angle of about 70 to 120 degrees. In some embodiments, the second pre-formed curve is bendable to an angle of about 10 to 50 degrees. In some embodiments, the distal section has a length of about 5 to 15 cm (about 2 to 6 inches), with the first pre-formed curve being formed in the proximal most 2.5 to 7.6 cm (1 to 3 inches), and the second pre-formed curve being formed in the distal most 2.5 to 7.6 cm (1 to 3 inches). In some embodiments, the first pre-formed curve is pre-curved to an angle of about 30 to about 80 degrees. In some embodiments, the second pre-formed curve is pre-curved to an angle of about 5 to about 30 degrees.

In some embodiments, the delivery catheter includes an outer jacket of a braided material. The outer jacket at the proximal section may include two layers while the outer jacket at the distal section has a single layer. In some embodiments, the proximal section has a greater hardness rating than the distal section. For example, a catheter wall of the proximal section may have a durometer of about 60D to about 90D, while a catheter wall of the distal section may have a durometer of about 35D to about 55D.

In some embodiments, at least the distal section of the delivery catheter includes a region of preferential bending, where one side of the distal section is formed from a relatively less stiff material and the opposite side of the distal section is formed from a relatively stiffer material.

An intermediate coupler is disposed at the distal section at a location proximal of the distal coupler. One or more intermediate control lines, each operatively coupled to a control of the handle, extend to engage with the intermediate coupler. These intermediate control lines enable manipulation of the intermediate coupler. Manipulation of the intermediate coupler provides control of the first pre-formed curve and manipulation of the distal coupler provides control of the second pre-formed curve.

Additional features and advantages will be set forth in part in the description that follows, and in part will be obvious from the description, or may be learned by practice of the arrangements disclosed herein. It is to be understood that both the foregoing brief summary and the following detailed description are exemplary and explanatory only and are not restrictive of the embodiments disclosed herein or as claimed.

### BRIEF DESCRIPTION OF THE DRAWINGS

In order to describe various features and concepts of the present disclosure, a more particular description of certain subject matter will be rendered by reference to specific arrangements which are illustrated in the appended drawings. Understanding that these figures depict examples and are not to be considered to be limiting in scope, various arrangements will be described and explained with additional specificity and detail through the use of the accompanying drawings in which:
Figure 1 illustrates a delivery system showing delivery of an interventional device through a delivery catheter to a position beyond the distal end of the delivery catheter;
Figure 2 illustrates a human heart and shows an exemplary intravascular approach by which the delivery catheter may be routed to the heart;
Figure 3 illustrates a perspective view of an exemplary delivery system showing a handle and a delivery catheter coupled to the handle;
Figure 4 illustrates an expanded view of the distal section of the delivery catheter, showing an exemplary coupler and control mechanism for manipulating the coupler;
Figure 5 illustrates the delivery catheter in a position superior to the mitral valve, showing a compound curve formed in the delivery catheter to enable the illustrated orientation;
Figures 6 and 7 illustrate cross-sectional views of a proximal section and a distal section of the delivery catheter, respectively.
Figure 8 illustrates another example of a delivery system, the illustrated example including a delivery catheter with an intermediate coupler;
Figure 9 illustrates an expanded view of the distal section of the delivery catheter shown in Figure 8, showing the intermediate coupler, a distal coupler, and a control mechanism for manipulating the intermediate and distal couplers; and
Figure 10 illustrates the delivery catheter of Figure 8 in a position superior to the mitral valve, showing a compound curve formed in the delivery catheter to enable the illustrated orientation;

### DETAILED DESCRIPTION

The present disclosure relates to intravascular delivery systems having centralized steering. The centralized steering features described herein provide effective routing of the delivery system to a targeted treatment site within a patient's vasculature. In contrast to conventional intravascular delivery systems, the arrangements described herein provide effective steering with minimized complexity and a simplified control scheme. For example, many conventional delivery systems require multiple radially nested catheters that must be independently controlled and positioned to provide the steering capabilities necessitated by a particular procedure. Arrangements described herein centralize steering functionality into an integrated elongated member and therefore minimize the number of required system parts, the number of separate delivery steps, and the concomitant risks of mechanical failure and/or human error.

The centralized steering features described herein may also reduce operator error associated with assembly of the intravascular delivery system. For example, an operator may be required to pre-assemble a conventional delivery system by positioning one or more internal guide catheters within an outer guide catheter. If the multiple guide catheters are not properly keyed (axially and rotationally) when they are nested together, the delivery system will be unlikely to function properly during the procedure. In contrast, delivery system arrangements described herein reduce or eliminate the need for such pre-assembly, and therefore minimize the potential for such errors and their associated patient risks.

The targeted treatment site to which a delivery system is delivered may include, for example, a regurgitant/malfunctioning cardiac valve. Delivery systems described herein may be routed to the targeted treatment site to enable delivery of one or more interventional devices through the delivery system and to the treatment site. Such interventional devices may include, for example, a cardiac valve repair device (e.g., such as the MitraClip^{®} available from Abbott Vascular), a replacement valve, a chordae replacement, an annuloplasty device, an occluder, or other interventional tool which may be utilized in an intravascular treatment procedure.

Throughout this disclosure, many examples are described in the context of guiding a delivery system to a mitral valve. One of skill in the art will understand, however, that the described components, features, and principles may also be utilized in other applications. For example, at least some of the arrangements described herein may be utilized for guiding a delivery system to a pulmonary, aortic, or tricuspid valve.

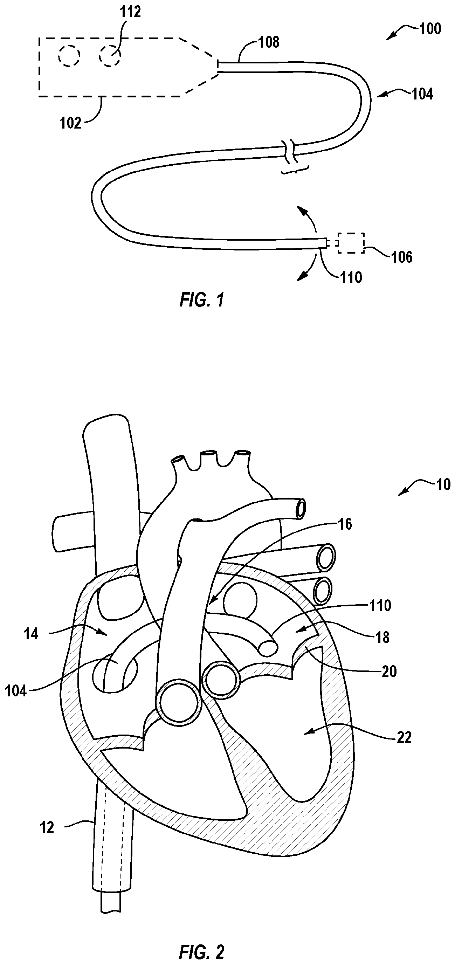

Figure 1 illustrates a delivery system 100 having a handle 102 and a delivery catheter 104 coupled to the handle 102. The handle 102 is connected to the proximal end 108 of the catheter 104 and may be configured to be operatively connected to one or more lumens of the catheter 104 to provide movement control over the catheter 104. An interventional device 106 may be connected to a distal end 110 of the catheter 104 or may be passable through an inner lumen of the catheter 104 and out through the distal end 110. One or more controls 112 may be included at the handle 102. The one or more controls 112 may be operatively coupled to the delivery catheter 104 (e.g., to provide steering control) and/or to the interventional device 106 (e.g., to provide deployment/actuation control).

Figure 2 illustrates a schematic representation of a patient's heart and a medical procedure that may be conducted using a delivery system according to the present disclosure. The delivery catheter 104 may be inserted into the patient's vasculature and directed to the inferior vena cava 12. The catheter 104 is passed through the inferior vena cava 12 toward the heart. Upon entering the heart from the inferior vena cava 12, the catheter 104 enters the right atrium 14. For procedures associated with the mitral valve 20, such as deployment of a repair clip, the catheter 104 must further pass into the left atrium 18. As shown, the catheter 104 may reach the left atrium 18 through a puncture in the intra-atrial septum 16.

In other implementations, such as for procedures associated with a tricuspid valve, the catheter 104 may be passed through the inferior vena cava 12 into the right atrium 14, where it may then be positioned and used to perform the procedure related to the tricuspid valve. As described above, although many of the examples described herein are directed to the mitral valve, one or more arrangements may be utilized in other cardiac procedures, including those involving the tricuspid valve.

To perform an intravascular maneuver such as that shown in Figure 2, the delivery catheter 104 must be appropriately steered to the targeted treatment location. Precise control of the delivery catheter 104, as provided by one or more of the arrangements described herein, may provide effective positioning of the interventional device 106. For example, the delivery catheter 104 may be brought to a desired position and orientation, after which one or more interventional devices 106 may be passed through the delivery catheter 104 and guided to the targeted treatment site.

Although Figure 2 and many of the other examples described herein refer to a transfemoral approach for accessing a targeted treatment site, it will be understood that the arrangements described herein may also be utilized where alternative approaches are used. For example, arrangements described herein may be utilized in a transjugular approach, transapical approach, or other suitable approach.

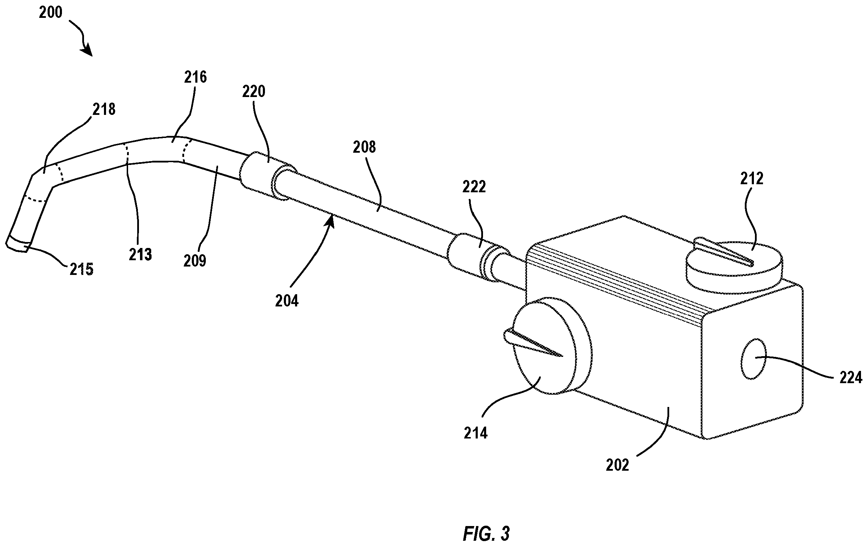

Figure 3 illustrates an exemplary arrangement of a delivery system 200 having centralized steering. The delivery system 200 may be utilized in the same general manner as the delivery system 100 described in relation to Figures 1 and 2, and is shown here separately to better illustrate additional features and components of the system.

As shown, a handle 202 is coupled to a delivery catheter 204. The delivery catheter 204 includes a proximal section 208 and a distal section 209. The proximal section 208 and distal section 209 may be attached by a joint 220. The joint 220 may be formed as an adhesive weld, a fitting (external and/or internal), or other suitable fastening structure. In alternative arrangements, the proximal section 208 and distal section 209 may be co-extruded as an integrally joined piece of material. The delivery system 200 may also include a valve 222 for managing fluid flows to and/or from the targeted treatment site. The delivery catheter 204 may also include one or more radiopaque markers 213 for imaging/tracking purposes.

The illustrated handle 202 includes an inlet 224 for providing access to the interior of the delivery catheter 204. The inlet 224 allows an interventional tool to be coupled to the delivery system 200 to be passed, for example, through the delivery catheter 204 to a targeted treatment site. The handle 202 also includes controls 212 and 214 which are operatively coupled to the delivery catheter 204. The controls 212 and 214 are configured to enable an operator to control steering of the delivery catheter 204 through manipulation of the controls 212 and 214. The controls 212 and 214 are shown here as turn knobs. However, it will be understood that other suitable controls, such as buttons, sliders, switches, and the like, may additionally or alternatively be utilized. Additional examples of handles and other delivery system components which may be utilized with the delivery catheter arrangements described herein are described in U.S. Patent Application Publication No. 2017/0100250.

The distal section 209 is curvable to enable steering and proper orientation of the distal end 215 relative to a treatment site. The distal section 209 is configured to form a plurality of separate curves such that a resulting compound curve can provide desired positioning and orientation of the distal end 215. As shown, the distal section 209 may be configured to form a first curve 216 at a proximal portion of the distal section 209, and a second curve 218 at a more distal portion of the distal section 209.

The first curve 216 and the second curve 218 preferably lie in different planes, though in some arrangements, they may lie in the same plane. In the illustrated example, the second curve 218 lies on a plane that is substantially orthogonal to the plane on which the first curve 216 lies. For example, the first curve 216 may be configured such that, when the distal section 209 is positioned within a patient's heart (e.g., in the manner illustrated in Figure 2), the first curve 216 lies substantially within the patient's coronal plane. Manipulation of the first curve 216 can thereby control positioning of the distal end 215 in the medial and lateral directions. Likewise, the second curve 218 may be configured such that, when the distal section 209 is positioned within a patient's heart (e.g., in the manner illustrated in Figure 2), the second curve 218 lies substantially within the patient's sagittal plane. Manipulation of the second curve 218 can thereby control positioning of the distal end 215 in the anterior and posterior directions.

In alternative arrangements, the first and/or second curves 216 and 218 may be configured with a different orientation. For example, the first curve 216 may be configured to substantially lie within the sagittal plane while the second curve 218 is configured to substantially lie within the coronal plane when deployed within a patient. In other arrangements, one or more of the first curve 216 or second curve 218 may lie within a plane disposed between the patient's coronal plane and sagittal plane. The relative positions of the first and second curves 216 and 218 may be configured according to particular patient anatomy and/or application needs.

The compound curve formable in the distal section 209 beneficially provides access to targeted intravascular treatment sites, such as the mitral valve. In preferred arrangements, the first curve 216 is configured so as to be bendable to an angle of about 70 to 120 degrees, or about 95 degrees. The second curve 218 may be configured to be bendable to an angle of about 10 to 50 degrees, or about 30 degrees. Compound curves formed using first and second curves within the foregoing angular ranges are particularly useful for accessing a mitral valve treatment site (e.g., through an approach as illustrated in Figure 2).

Exemplary control mechanisms for controlling the curvature of the first curve 216 and second curve 218 are described in more detail below. To facilitate curvature along desired planes and/or in desired directions, the first curve 216 and/or second curve 218 are pre-curved. As used herein "pre-curved" means that the relevant section of the delivery catheter 204 has a default or natural position, when not subjected to an overriding bending force, which is curved. Such pre-curved sections are not necessarily always curved. For example, while routing the delivery catheter 204 through a patient's vasculature, a pre-curved section may be substantially straightened or otherwise lessened in curvature.

In some arrangements, the first curve 216 is pre-curved to an angle of about 30 to 80 degrees, or about 45 to 65 degrees, or about 55 degrees. In some arrangements, the second curve 218 is pre-curved to an angle of about 5 to 30 degrees, or about 10 to 20 degrees. A distal section 209 having one or more pre-curved sections within the foregoing ranges is particularly useful for providing access to a mitral valve treatment site. For example, where the first curve 216 and/or 218 second curve are pre-curved to a level such as within the foregoing ranges, an operator may readily and effectively route the distal section 209 to the targeted site, and may then augment the curvature(s) to provide a desired orientation with respect to the targeted site. In one example, the pre-curved first section 216 is further curvable by up to about 70 degrees, and/or the second curve is further curvable by up to about 30 degrees.

The delivery catheter 204 may have any length appropriate for accessing the targeted treatment site. Generally, the overall length of the delivery catheter 204 is about 102 cm to 203 cm (about 40 to 80 inches), more commonly about 152 cm (about 60 inches). The distal section 209 may have a length of about 5 cm to 15 cm (about 2 to 6 inches), with the first curve 216 being formed in the proximal most 2.5 to 7.6 cm (1 to 3 inches) (e.g., about 3.8 cm (about 1.5 inches)) of the distal section 209 and the second curve 218 being formed in the distal most 2.5 to 7.6 cm (1 to 3 inches)(e.g., about 3.8 cm about 1.5 inches)) of the distal section 209.

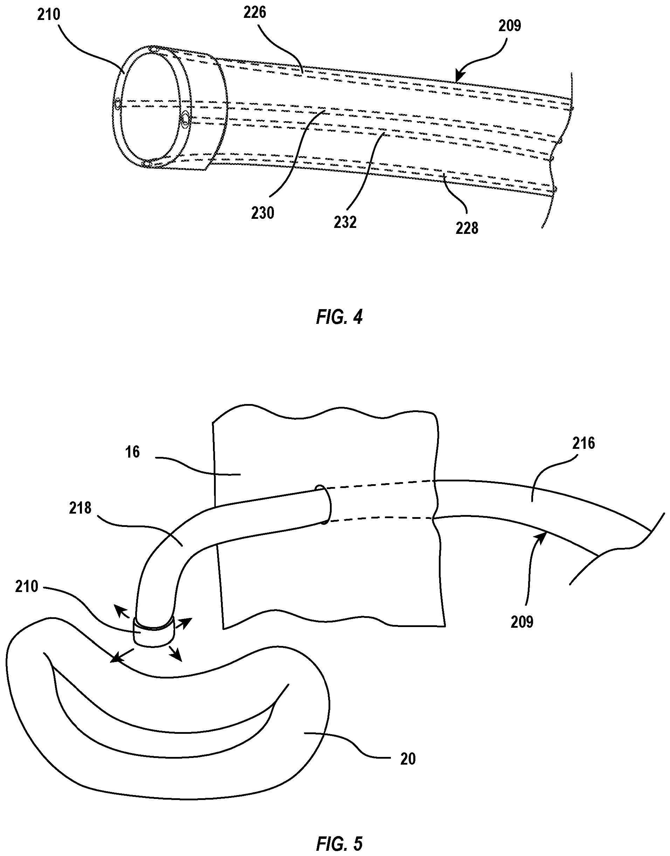

Figure 4 illustrates an exemplary control mechanism which may be utilized to steer the delivery catheter 204. Figure 4 shows an expanded view of the distal section 209 of the delivery catheter 204. In the illustrated arrangement, a coupler 210 is disposed at the distal end of the catheter. A plurality of control lines 226, 228, 230, and 232 extend through the interior of the catheter from the handle 202 (see Figure 3) and are attached to the coupler 210. Selective application of tension to one or more of the control lines causes the catheter to curve in the direction of the tensioned control line(s), enabling an operator to control steering of the distal section 209 through manipulation of one or more of the control lines 226, 228, 230, and 232.

In the illustrated arrangement, the control lines 226, 228, 230, and 232 are operatively coupled to the handle controls 212 and 214 (see Figure 3) such that an operator can control tension in the control lines 226, 228, 230, and 232 through manipulation of the handle controls 212 and 214. In some arrangements, each opposing pair of control lines are coupled to one of the handle controls. For example, control lines 226 and 228, which are positioned circumferentially opposite one another within the delivery catheter, may both be coupled to one of the controls (e.g., control 212), while control lines 230 and 232, which are positioned circumferentially opposite one another within the delivery catheter, may both be coupled to the other control (e.g., control 214).

In this exemplary configuration, manipulation of control 212 adjusts curvature along the plane defined by control lines 226 and 228, while manipulation of control 214 adjusts curvature along the plane defined by control lines 230 and 232. The plane defined by control lines 226 and 228 may be the plane on which the first curve 216 lies, while the plane defined by control lines 230 and 232 may be the plane on which the second curve 218 lies.

Although the illustrated delivery systems are shown as having two handle controls, it will be understood that other numbers of controls may be utilized in alternative arrangements. Some arrangements may include one or more additional controls. In one example, each control line in a pair of corresponding, circumferentially opposed control lines may be operatively coupled to a separate control at the handle 202. For example, control line 226 and 228 may be coupled to separate controls at the handle 202 rather than to the same control.

The coupler 210 is shown in the illustrated arrangement as a ring structure configured to receive and engage with the control lines 226, 228, 230, and 232. In alternative arrangements, the coupler 210 may be configured as a band, fitting, or other suitable structure for receiving and engaging with control lines 226, 228, 230, and 232. In other arrangements, one or more control lines may be affixed to the catheter using an adhesive.

In the illustrated arrangement, each control line 226, 228, 230, and 232 pass through the catheter to the coupler 210 and then loop back to pass back toward the handle 202. Such a double-strand configuration provides for effective tensioning of the control lines with reduced risk of line breakage or detachment. Alternatively, one or more control lines may be welded to the coupler 210 and need not necessarily loop back toward the handle 202. Additional examples of ring structures which may be utilized as coupler 210 or as other coupler arrangements taught herein are described in U.S. Patent Application Publication No. 2016/0367787.

Figure 5 illustrates the distal section 209 of the delivery catheter 204 in a position superior to the mitral valve 20 after having passed through the septum 16. As shown, manipulation of a first set of circumferentially opposed control lines (e.g., control lines 226 and 228) can form and/or adjust the first curve 216 while manipulation of a second set of circumferentially opposing control lines (e.g., control lines 230 and 232) can form and/or adjust the second curve 218. The resulting compound curve allows the delivery catheter 204 to be oriented at a desired location relative to the targeted mitral valve 20.

Although all of the control lines 226, 228, 230, and 232 terminate at the same coupler 210, the curves 216 and 218 may be primarily formed at different regions of the distal section 209 as a result of pre-curvature and, optionally, also preferential bending features at the different regions. For example, pre-curvature and, optionally, also preferential bending features of the delivery catheter 204 (e.g., particularly at the distal section 209) may promote curvature associated with manipulation of a first pair of control wires to occur primarily at the first curve 216, and curvature associated with manipulation of a second pair of control wires to occur primarily further distally at the second curve 218.

Once positioned at a desired location relative to the targeted mitral valve 20, one or more interventional devices may be delivered through the delivery catheter 204 in order to perform the desired repair or replacement procedure. Further adjustment of the delivery catheter position may also be done intra-procedurally as desired.

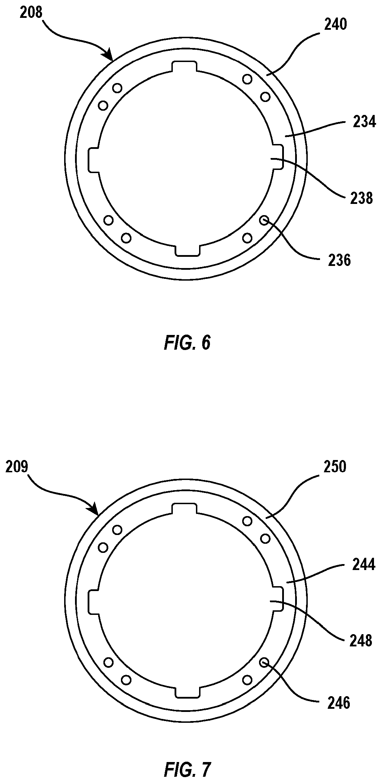

Figures 6 and 7 illustrate cross-sectional views of the proximal section 208 and distal section 209 of the delivery catheter 204, respectively. As shown in Figure 6, the proximal section 208 includes a catheter wall 234 surrounded by a jacket 240. The jacket 240 may be formed from one or more layers of a reinforcing material. In some arrangements, the one or more layers of reinforcing material include a braided material, such as a braided metal or polymer material. In preferred arrangements, the jacket 240 of the proximal section 208 includes two layers of braided material. The braided material preferably has a pic count of about 10 to 50, such as relatively higher pic counts of about 30 to 50.

In some arrangements, the catheter wall 234 is formed from a medical-grade polymer such as polyether block amide (e.g., Pebax^{®}), polyurethane, nylon, polyethylene, polyimide, polyethylene terephthalate (PET), or polyetheretherketone (PEEK). Preferably, the catheter wall 234 of the proximal section 208 has a hardness of greater than about 35D (e.g., using the Shore D scale) and up to about 90D. For example, in some arrangements the catheter wall 234 may be formed from Pebax having a durometer of above 60D to about 75D. In some arrangements, the catheter wall 234 may be formed from a nylon material (e.g., Nylon-12) having a durometer of about 80D to 90D.

The components of the proximal section 208 are configured to provide effective column strength and torque transmissibility. Although the proximal section 208 may include pre-shaping, in preferred arrangements the proximal section 208 omits pre-shaping. The proximal section 208 is preferably instead configured to provide column strength and torque transmissibility so that the distal section 209 can be effectively delivered to the targeted treatment site.

The catheter wall 234 may include one or more wall lumens 236. In the illustrated arrangement, four pairs of wall lumens 236 are disposed at approximately 90 degree increments around the cross-section of the proximal section 208. Each pair is configured to channel a control line from the handle 202 to the coupler 210 (see Figure 4) and back to the handle 202. In each pair, one lumen may be used to route the control line from the handle 202 to the coupler 210, while the other lumen may be used to route the control line back to the handle after looping around the coupler 210. In alternative arrangements, other lumen arrangements may be utilized for directing one or more control lines between the handle 202 and more distal sections of the delivery catheter. For example, a control line may pass from the handle 202 to the coupler 210 and then back to the handle 202 with both strands passing through the same wall lumen.

The illustrated arrangement also includes a set of keyways 238. One or more of such keyways 238 may be included for rotational keying of the proximal section 208 with an interventional device passed through the inner lumen. For example, the interventional device may include one or more keys or grooves configured to match the keyways 238 to rotationally align the interventional tool with the proximal section 208. Alternative arrangements additionally or alternatively use other keying features. For example, the catheter wall 234 may include one or more keys, tabs, or grooves configured to match with a corresponding keyway of the interventional device.

Figure 7 illustrates a cross-sectional view of the distal section 209. As shown, the distal section 209 may have substantially the same profile and general dimensions (e.g., inner diameter and outer diameter). Like the proximal section 208, the distal section 209 includes a catheter wall 244 and an outer jacket 250, and may also include one or more wall lumens 246 and keyways 248. The above discussion related to the proximal section 208 is applicable to the corresponding components of the distal section 209. However, the catheter wall 244 of the distal section 209 is preferably less stiff than the catheter wall 234 of the proximal section 208. For example, the catheter wall 244 may have a durometer of about 35D to 75D.

The catheter wall 244 of the distal section 209 may be formed from the same material as the catheter wall 234 of the proximal section 208 or may be formed from a different material. For example, the catheter wall 234 and the catheter wall 244 may both be formed from Pebax, with the Pebax of the catheter wall 234 being a harder grade than the Pebax of the catheter wall 244. In another example, the catheter wall 234 is formed from Nylon-12 and the catheter wall 244 is formed from a relatively softer Pebax.

As described above, the distal section 209 is formed with one or more regions of pre-curvature and, optionally, also preferential bending. In some arrangements, such a region is formed by co-extruding the catheter wall 244 with materials of differing stiffness. For example, one side (e.g., about 50%) of the extruded catheter wall 244 may be extruded or otherwise formed with a material of relatively higher stiffness while the opposite side is extruded or otherwise formed with a material of relatively lower stiffness. In some arrangements, a relatively harder grade of Pebax or other suitable material (e.g., about 45D to 55D) is utilized for the higher stiffness side and a softer grade of Pebax or other suitable material (e.g., about 35D to 45D) is utilized for the lower stiffness side. The side formed of the lower stiffness material may then function as a direction of preferred bending. Heat setting or other suitable method may be utilized to set a pre-shaped curve.

The jacket 250 of the distal section 209 may have a lower pic count and/or less layers as compared to the jacket 240 of the proximal section 208. For example, the jacket 240 may utilize a single layer of braided material having a pic count of about 10 to 30.

The components of the distal section 209 are configured to provide effective steerability to the distal section 209, while the proximal section 208 is generally configured to provide effective torquability and column strength. In combination, the proximal section 208 and distal section 209 therefore provide, as a single integrated catheter, effective routing to a targeted treatment site and effective steerability and maneuverability of the distal tip relative to the targeted treatment site

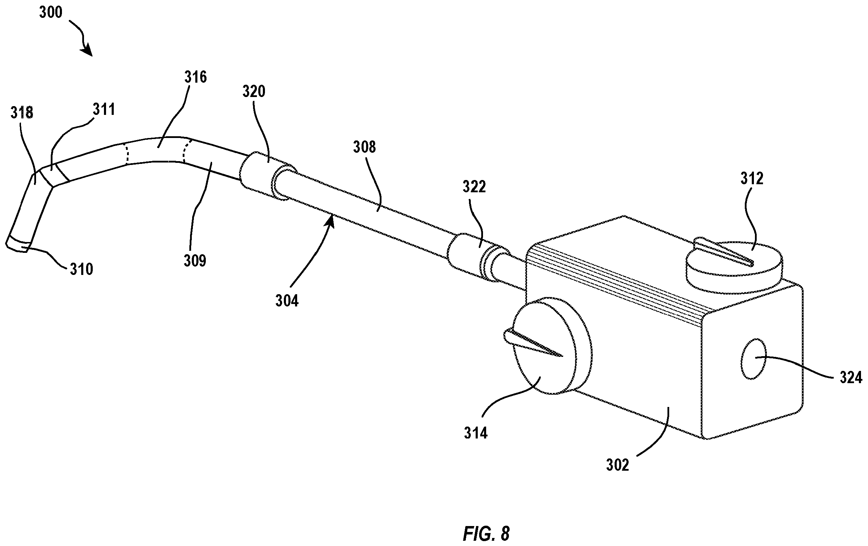

Figures 8 through 10 illustrate an alternative arrangement of a delivery system 300 which may be utilized to deliver one or more interventional devices in an intravascular procedure. The arrangement shown in Figures 8 through 10 is similar in many aspects to the arrangement illustrated in Figures 2 through 7. The previous discussion related to delivery system 200 is therefore likewise applicable to delivery system 300, except where differences are expressly noted below.

As shown in Figure 8, the delivery system 300 includes a handle 302 with an inlet 324 and controls 312 and 314. The handle 302 is coupled to a delivery catheter 304 and may include a valve 322 for managing fluid flow to and/or from the targeted treatment site. The delivery catheter 304 includes a proximal section 308 and a distal section 309. The proximal section 308 and distal section 309 may be coupled by a joint 320.

The distal section 309 of the illustrated arrangement includes a distal coupler 310 disposed at the distal end of the delivery catheter 304. The distal coupler 310 may be configured in a manner similar to the coupler 210 of delivery system 200 as described above. In addition to the distal coupler 310, the illustrated delivery catheter 304 further includes an intermediate coupler 311 disposed a distance proximal of the distal coupler 310. The intermediate coupler 311 and distal coupler 310 function to enable control of the curvatures of the first curve 316 and second curve 318, respectively.

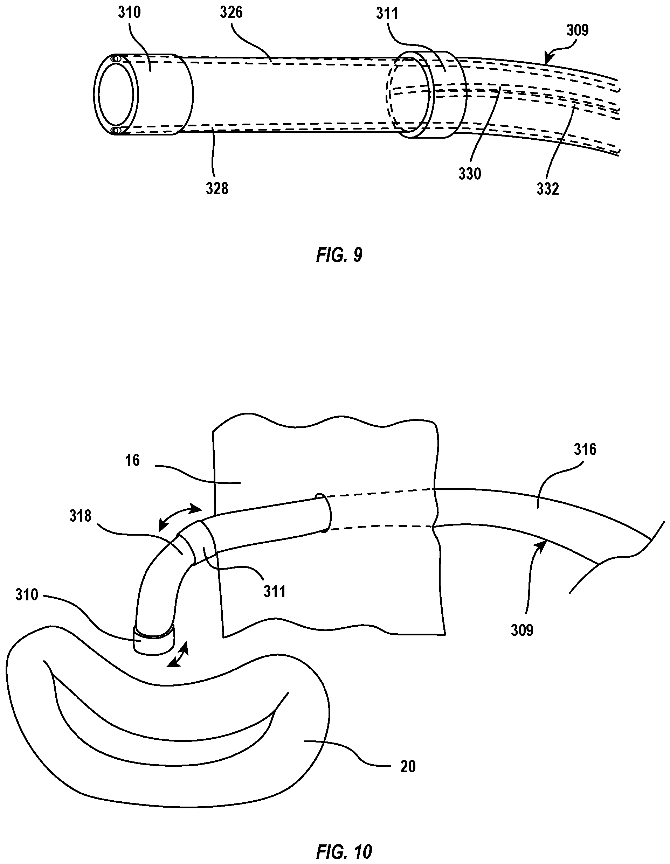

Figure 9 illustrates an expanded view of a portion of the distal section 309. As shown, a series of control lines 326, 328, 330, and 332 extend to the intermediate coupler 311. The control lines 326, 328, 330, and 332 may extend from the handle 302, for example, and may be operatively coupled to the controls 312 and 314 in a manner similar to that described above in relation to delivery system 200 and its similar components. In the arrangement shown in Figure 9, control lines 330 and 332 terminate at the intermediate coupler 311 before passing back toward the handle 302, while control lines 326 and 328 continue distally to distal coupler 310 before passing back toward the handle 302. In some arrangements, the intermediate coupler 311 may include corresponding lumens through which the control lines 326 and 328 may pass.

In the illustrated arrangement, the intermediate coupler 311 is positioned such that manipulation of control lines 330 and 332 moves the intermediate coupler 311 and thereby adjusts the first curve 316. Similarly, the distal coupler 310 is positioned such that manipulation of control lines 326 and 328 moves the distal coupler 310 and thereby adjusts the second curve 318.

In the illustrated arrangement, control lines 326 and 328 are positioned circumferentially opposite one another and control lines 330 and 332 are positioned circumferentially opposite one another. Each pair of control lines may respectively be coupled to a different control 312 and 314, such that one control functions to adjust the intermediate coupler 311 and first curve 316 (e.g., along a plane defined by control lines 330 and 332) while the other control functions to adjust the distal coupler 310 and second curve 318 (e.g., along a plane defined by control lines 326 and 328). The plane on which the first curve 316 lies may be orthogonal to the plane on which the second curve 318 lies. Alternative arrangements may position one or more control lines differently so as to define different planes of curvature for the first curve 316 and/or second curve 318.

Figure 10 illustrates the distal section 309 of the delivery catheter 304 in a position superior to the mitral valve 20 after having passed through the septum 16. As shown, manipulation of a first set of circumferentially opposed control lines (e.g., control lines 330 and 332) can form and/or adjust the first curve 316 by applying tension to the intermediate coupler 311. Likewise, manipulation of a second set of circumferentially opposed control lines (e.g., control lines 326 and 328) can form and/or adjust the second curve 318 by applying tension to the distal coupler 310. The resulting compound curve allows the delivery catheter 304 to be oriented at a desired location relative to the targeted mitral valve 20.

The proximal section 308 and distal section 309 of the illustrated arrangement may have cross-sectional configurations, materials, and pre-curvature and/or preferential bending features similar to those of corresponding components of delivery system 200, such as shown in Figures 6 and 7 and discussed in the corresponding description.

The articles "a," "an," and "the" are intended to mean that there are one or more of the elements in the preceding descriptions. The terms "comprising," "including," and "having" are intended to be inclusive and mean that there may be additional elements other than the listed elements. Additionally, it should be understood that references to "one arrangement" or "an arrangement" are not intended to be interpreted as excluding the existence of additional arrangements that also incorporate the recited features. Numbers, percentages, ratios, or other values stated herein are intended to include that value, and also other values that are "about" or "approximately" the stated value, as would be appreciated by one of ordinary skill in the art encompassed by the present disclosure. A stated value should therefore be interpreted broadly enough to encompass values that are at least close enough to the stated value to perform a desired function or achieve a desired result. The stated values include at least the variation to be expected in a suitable manufacturing or production process, and may include values that are within 5%, within 1%, within 0.1%, or within 0.01% of a stated value.

Elements described in relation to any arrangement depicted and/or described herein may be substituted for or combined with elements described in relation to any other arrangement depicted and/or described herein. For example, any of the components or features described in relation to delivery system 200 may be substituted for or combined with any of the components or features described in relation to delivery system 300, and vice versa.

## Claims

1. A delivery system (300) for delivery of an interventional device (106) in an intravascular procedure, the delivery system (300) comprising:
a handle (302) having one or more controls (312, 314);
a delivery catheter (304) having a proximal end and a distal end, the proximal end being coupled to the handle (302), the delivery catheter (304) having a proximal section (308) and a distal section (309),
wherein at least the distal section (309) is configured to selectively form a compound curve to enable positioning of the distal end relative to a targeted intravascular treatment site, and
wherein the compound curve includes a first pre-formed curve (316) at a proximal portion of the distal section (309) and a second pre-formed curve (318) at a distal portion of the distal section (309);
a series of control lines (326, 328, 330, 332), each operatively coupled to a control (312, 314) of the handle (302) and each extending from the handle (302) through a wall lumen of the delivery catheter (304) toward the distal end;
a distal coupler (310) disposed at or near the distal end of the delivery catheter (304), one or more control lines (326, 328) extending to the distal coupler (310) and engaging with the distal coupler (310) such that tensioning of the one or more control lines (326, 328) changes position of the distal coupler (310) to thereby form or adjust the compound curve; and
an intermediate coupler (311) disposed at the distal section (309) at a location proximal of the distal coupler (310), and further comprising one or more intermediate control lines (330, 332), each operatively coupled to a control (312) of the handle (302) and extending to engage with the intermediate coupler (311) and to enable manipulation of the intermediate coupler (311), manipulation of the intermediate coupler (311) thereby controlling the first pre-formed curve (316) and manipulation of the distal coupler (310) thereby controlling the second pre-formed curve (318).

2. The delivery system (300) of claim 1, wherein the first pre-formed curve (316) and second pre-formed curve (318) lie in different planes.

3. The delivery system (300) of claim 2, wherein the first pre-formed curve (316) lies in a plane that is substantially orthogonal to a plane in which the second pre-formed curve (318) lies.

4. The delivery system (300) of claim 1, wherein the first pre-formed curve (316) is configured to be bendable to an angle of about 70 to about 120 degrees.

5. The delivery system (300) of claim 1, wherein the second pre-formed curve (318) is configured to be bendable to an angle of about 10 to about 50 degrees.

6. The delivery system (300) of claim 1, wherein the distal section (309) has a length of about 5 to 15 cm (about 2 to 6 inches), with the first pre-formed curve (316) being formed in the proximal most 2.5 to 7.6 cm (1 to 3 inches), and the second pre-formed curve (318) being formed in the distal most 2.5 to 7.6 cm (1 to 3 inches).

7. The delivery system (300) of claim 1, wherein the first pre-formed curve (316) is pre-curved to an angle of about 30 to about 80 degrees.

8. The delivery system (300) of claim 1, wherein the second pre-formed curve (318) is pre-curved to an angle of about 5 to about 30 degrees.

9. The delivery system (300) of claim 1, wherein each control line (326, 328) extending to the distal coupler (310) extends from a control (314) of the handle (302) to the distal coupler (310) and then loops back toward the handle (302).

10. The delivery system (300) of claim 1, comprising a first pair of control lines (326, 328) circumferentially opposed to one another at the intermediate coupler (311), and a second pair of control lines (330, 332) circumferentially opposed to one another at the intermediate coupler (311), the first pair (326, 328) and the second pair (330, 332) being offset by about 90 degrees.

11. The delivery system (300) of claim 1, wherein the delivery catheter (304) includes an outer jacket of a braided material.

12. The delivery system (300) of claim 11, wherein the outer jacket at the proximal section (308) comprises two layers and at the distal section (309) has a single layer.

13. The delivery system (300) of claim 1, wherein the proximal section (308) has a greater hardness rating than the distal section (309).

14. The delivery system (300) of claim 13, wherein a catheter wall of the proximal section (308) has a durometer of about 60D to about 90D, and wherein a catheter wall of the distal section (309) has a durometer of about 35D to about 55D.

15. The delivery system (300) of claim 1, wherein at least the distal section (309) includes a region of preferential bending, wherein one side of the distal section (309) is formed from a relatively less stiff material and the opposite side of the distal section (309) is formed from a relatively stiffer material.

## Patentansprüche

1. Abgabesystem (300) zum Abgeben einer chirurgischen Vorrichtung (106) in einem intravaskulären Verfahren, wobei das Abgabesystem (300) umfasst:
einen Griff (302) mit einem oder mehreren Bedienelementen (312, 314);
einen Abgabekatheter (304) mit einem proximalen Ende und einem distalen Ende, wobei das proximale Ende mit dem Handgriff (302) verbunden ist und der Abgabekatheter (304) einen proximalen Abschnitt (308) und einen distalen Abschnitt (309) aufweist,
wobei zumindest der distale Abschnitt (309) konfiguriert ist, um selektiv eine zusammengesetzte Krümmung zu bilden, um eine Positionierung des distalen Endes relativ zu einer angepeilten intravaskulären Behandlungsstelle zu ermöglichen, und
wobei die zusammengesetzte Krümmung eine erste vorgeformte Krümmung (316) an einem proximalen Teil des distalen Abschnitts (309) und eine zweite vorgeformte Krümmung (318) an einem distalen Teil des distalen Abschnitts (309) aufweist;
eine Reihe von Steuerleitungen (326, 328, 330, 332), die jeweils funktionsmäßig mit einer Steuerung (312, 314) des Griffs (302) gekoppelt sind und sich jeweils von dem Griff (302) durch ein Wandlumen des Abgabekatheters (304) zu dem distalen Ende hin erstrecken;
eine distale Kupplung (310), die am oder in der Nähe des distalen Endes des Abgabekatheters (304) angeordnet ist, wobei sich eine oder mehrere Steuerleitungen (326, 328) zu der distalen Kupplung (310) erstrecken und mit der distalen Kupplung (310) in Eingriff stehen, so dass das Spannen der ein oder mehreren Steuerleitungen (326, 328) die Position der distalen Kupplung (310) ändert, um dadurch die zusammengesetzte Krümmung zu bilden oder anzupassen; und
eine Zwischenkupplung (311), die an dem distalen Abschnitt (309) an einer Stelle proximal zu der distalen Kupplung (310) angeordnet ist und die ferner ein oder mehrere Zwischensteuerleitungen (330, 332) umfasst, die jeweils funktionsmäßig mit einer Steuerung (312) des Griffs (302) gekoppelt sind und sich so erstrecken, dass sie mit der Zwischenkupplung (311) in Eingriff kommen und eine Manipulation der Zwischenkupplung (311) ermöglichen, wobei die Manipulation der Zwischenkupplung (311) dadurch die erste vorgeformte Krümmung (316) steuert und die Manipulation der distalen Kupplung (310) dadurch die zweite vorgeformte Krümmung (318) steuert.

2. Abgabesystem (300) nach Anspruch 1, bei dem die erste vorgeformte Krümmung (316) und die zweite vorgeformte Krümmung (318) in verschiedenen Ebenen liegen.

3. Abgabesystem (300) nach Anspruch 2, bei dem die erste vorgeformte Krümmung (316) in einer Ebene liegt, die im Wesentlichen orthogonal zu einer Ebene ist, in der die zweite vorgeformte Krümmung (318) liegt.

4. Abgabesystem (300) nach Anspruch 1, bei dem die erste vorgeformte Krümmung (316) konfiguriert ist, um in einem Winkel von etwa 70 bis etwa 120 Grad biegbar zu sein.

5. Abgabesystem (300) nach Anspruch 1, bei dem die zweite vorgeformte Krümmung (318) konfiguriert ist, um in einem Winkel von etwa 10 bis etwa 50 Grad biegbar zu sein.

6. Abgabesystem (300) nach Anspruch 1, bei dem der distale Abschnitt (309) eine Länge von etwa 5 bis 15 cm (etwa 2 bis 6 Zoll) hat, wobei die erste vorgeformte Krümmung (316) in den proximalsten 2,5 bis 7,6 cm (1 bis 3 Zoll) und die zweite vorgeformte Krümmung (318) in den distalsten 2,5 bis 7,6 cm (1 bis 3 Zoll) ausgebildet ist.

7. Abgabesystem (300) nach Anspruch 1, bei dem die erste vorgeformte Krümmung (316) in einem Winkel von etwa 30 bis etwa 80 Grad vorgekrümmt ist.

8. Abgabesystem (300) nach Anspruch 1, bei dem die zweite vorgeformte Krümmung (318) in einem Winkel von etwa 5 bis etwa 30 Grad vorgekrümmt ist.

9. Abgabesystem (300) nach Anspruch 1, bei dem jede Steuerleitung (326, 328), die sich zur distalen Kupplung (310) erstreckt, von einer Steuerung (314) des Griffs (302) zur distalen Kupplung (310) verläuft und dann in einer Schleife zurück zum Griff (302) führt.

10. Abgabesystem (300) nach Anspruch 1, umfassend ein erstes Paar von Steuerleitungen (326, 328), die sich an der Zwischenkupplung (311) in Umfangsrichtung gegenüberliegen, und ein zweites Paar von Steuerleitungen (330, 332), die sich an der Zwischenkupplung (311) in Umfangsrichtung gegenüberliegen, wobei das erste Paar (326, 328) und das zweite Paar (330, 332) um etwa 90 Grad versetzt sind.

11. Abgabesystem (300) nach Anspruch 1, bei dem der Abgabekatheter (304) einen Außenmantel aus einem geflochtenen Material aufweist.

12. Abgabesystem (300) nach Anspruch 11, bei dem der Außenmantel am proximalen Abschnitt (308) zwei Lagen umfasst und am distalen Abschnitt (309) eine einzige Lage aufweist.

13. Abgabesystem (300) nach Anspruch 1, bei dem der proximale Abschnitt (308) einen höheren Härtegrad aufweist als der distale Abschnitt (309).

14. Abgabesystem (300) nach Anspruch 13, bei dem eine Katheterwand des proximalen Abschnitts (308) einen Härtegrad von etwa 60D bis etwa 90D aufweist, und bei dem eine Katheterwand des distalen Abschnitts (309) einen Härtegrad von etwa 35D bis etwa 55D aufweist.

15. Abgabesystem (300) nach Anspruch 1, bei dem zumindest der distale Abschnitt (309) einen Bereich mit bevorzugter Biegung aufweist, wobei eine Seite des distalen Abschnitts (309) aus einem relativ weniger steifen Material und die gegenüberliegende Seite des distalen Abschnitts (309) aus einem relativ steiferen Material gebildet ist.

## Revendications

1. Système de mise en place (300) pour mettre en place un dispositif d'intervention (106) dans une procédure intravasculaire, le système de mise en place (300) comprenant :
une poignée (302) présentant une ou plusieurs commandes (312, 314) ;
un cathéter de mise en place (304) présentant une extrémité proximale et une extrémité distale, l'extrémité proximale étant couplée à la poignée (302), le cathéter de mise en place (304) présentant une section proximale (308) et une section distale (309),
dans lequel au moins la section distale (309) est configurée pour former sélectivement une courbe composite afin de permettre un positionnement de l'extrémité distale par rapport à un site de traitement intravasculaire ciblé, et
dans lequel la courbe composite inclut une première courbe préformée (316) au niveau d'une partie proximale de la section distale (309) et une seconde courbe préformée (318) au niveau d'une partie distale de la section distale (309) ;
une série de lignes de commande (326, 328, 330, 332), chacune couplée de manière fonctionnelle à une commande (312, 314) de la poignée (302) et chacune s'étendant à partir de la poignée (302) à travers une lumière de paroi du cathéter de mise en place (304) vers l'extrémité distale ;
un dispositif de couplage distal (310) disposé au niveau ou à proximité de l'extrémité distale du cathéter de mise en place (304), une ou plusieurs lignes de commande (326, 328) s'étendant vers le dispositif de couplage distal (310) et venant en prise avec le dispositif de couplage distal (310) de telle sorte qu'une mise en tension des une ou plusieurs lignes de commande (326, 328) change une position du dispositif de couplage distal (310) pour former ou ajuster de ce fait la courbe composite ; et
un dispositif de couplage intermédiaire (311) disposé au niveau de la section distale (309) à un emplacement proximal du dispositif de couplage distal (310), et comprenant en outre une ou plusieurs lignes de commande intermédiaires (330, 332), chacune étant couplée de manière fonctionnelle à une commande (312) de la poignée (302) et s'étendant pour venir en prise avec le dispositif de couplage intermédiaire (311) et pour permettre une manipulation du dispositif de couplage intermédiaire (311), la manipulation du dispositif de couplage intermédiaire (311) commandant ainsi la première courbe préformée (316) et la manipulation du dispositif de couplage distal (310) commandant ainsi la seconde courbe préformée (318).

2. Système de mise en place (300) selon la revendication 1, dans lequel la première courbe préformée (316) et la seconde courbe préformée (318) se trouvent dans des plans différents.

3. Système de mise en place (300) selon la revendication 2, dans lequel la première courbe préformée (316) se trouve dans un plan qui est sensiblement orthogonal à un plan dans lequel se trouve la seconde courbe préformée (318).

4. Système de mise en place (300) selon la revendication 1, dans lequel la première courbe préformée (316) est configurée pour être pliable selon un angle d'environ 70 à environ 120 degrés.

5. Système de mise en place (300) selon la revendication 1, dans lequel la seconde courbe préformée (318) est configurée pour pouvoir être pliée selon un angle d'environ 10 à environ 50 degrés.

6. Système de mise en place (300) selon la revendication 1, dans lequel la section distale (309) présente une longueur d'environ 5 à 15 cm (environ 2 à 6 pouces), la première courbe préformée (316) étant formée dans la plus proximale de 2,5 à 7,6 cm (1 à 3 pouces), et la seconde courbe préformée (318) étant formée dans la plus distale de 2,5 à 7,6 cm (1 à 3 pouces).

7. Système de mise en place (300) selon la revendication 1, dans lequel la première courbe préformée (316) est pré-courbée selon un angle d'environ 30 à environ 80 degrés.

8. Système de mise en place (300) selon la revendication 1, dans lequel la seconde courbe préformée (318) est pré-courbée selon un angle d'environ 5 à environ 30 degrés.

9. Système de mise en place (300) selon la revendication 1, dans lequel chaque ligne de commande (326, 328) s'étendant vers le dispositif de couplage distal (310) s'étend depuis une commande (314) de la poignée (302) vers le dispositif de couplage distal (310), puis fait une boucle vers l'arrière en direction de la poignée (302).

10. Système de mise en place (300) selon la revendication 1, comprenant une première paire de lignes de commande (326, 328) circonférentiellement opposées l'une à l'autre au niveau du dispositif de couplage intermédiaire (311), et une seconde paire de lignes de commande (330, 332) circonférentiellement opposées l'une à l'autre au niveau du dispositif de couplage intermédiaire (311), la première paire (326, 328) et la seconde paire (330, 332) étant décalées d'environ 90 degrés.

11. Système de mise en place (300) selon la revendication 1, dans lequel le cathéter de mise en place (304) inclut une enveloppe extérieure d'un matériau tressé.

12. Système de mise en place (300) selon la revendication 11, dans lequel l'enveloppe extérieure au niveau de la section proximale (308) comprend deux couches et présente une couche unique au niveau de la section distale (309) .

13. Système de mise en place (300) selon la revendication 1, dans lequel la section proximale (308) présente une plus grande dureté que la section distale (309).

14. Système de mise en place (300) selon la revendication 13, dans lequel une paroi de cathéter de la section proximale (308) présente une dureté d'environ 60D à environ 90D, et dans lequel une paroi de cathéter de la section distale (309) présente une dureté d'environ 35D à environ 55D.

15. Système de mise en place (300) selon la revendication 1, dans lequel au moins la section distale (309) inclut une région de pliure préférentielle, dans lequel un côté de la section distale (309) est formé d'un matériau relativement moins rigide et le côté opposé de la section distale (309) est formé d'un matériau relativement plus rigide.
